# EUROPEAN PATENT SPECIFICATION

(11) **EP 0 663 217 B1**
(45) Date of publication and mention of the grant of the patent: **15.09.1999**
(21) Application number: 95300119.5
(22) Date of filing: 10.01.1995
(51) Int. Cl.: A61M 25/00, A61B 17/32

(54) **Rotating tip with shroud for catheter**
Drehbarer Kopf mit Lager für einen Katheter
Pointe rotative avec bouclier pour cathéter

(30) Priority: 12.01.1994 US 180941
(43) Date of publication of application: 19.07.1995
(73) Proprietor: DOW CORNING ENTERPRISES, Midland, Michigan 48686-0994 (US)
(72) Inventor: Jaeger Jr., Cletus Wilfred, Sunrise, Florida (US); Wagner, Erik Joseph, Allen, Texas (US)
(74) Representative: Lewin, John Harvey

(56) References cited:
- EP-A- 0 291 170
- EP-A- 0 315 290

## Description

Due to a mechanism not entirely understood by physicians, the cardiovascular blood delivery system often becomes less efficient as one ages and results in arteriosclerosis and/or thrombosis. Although the causes of arteriosclerosis are not completely understood, one symptom is the partial or total blockage of a blood vessel due to a build up of deposits along an inner surface.

Several methods have been proposed for the opening of partially or totally blocked blood vessels. One such method is by inserting a balloon catheter within the vessel to expand the vessel and either break loose deposits within the vessel or alternatively, increase the size of the lumen passing through those deposits.

An alternative method is to bring a high speed device into contact with occluded portions of the blood vessel. The rotating device opens the vessel and increases blood flow by cutting, abrading, impacting or fluid turbulence to remove the obstructing material.

Under certain circumstances it is desirable to protect Surrounding bodily tissues from damage caused by the rotary motion of the blades by placing a shroud on the distal end of the catheter. This has previously been done by placing the shroud over the tip as a separate component from the catheter or bushing. For example, U.S. Patent 4,664,112 discloses a power driven catheter for intravascular surgery. Figures 11 and 12 therein show a shroud which is fixedly secured on the distal end of the catheter tube by a retaining band. U.S. Patent 4,811,735 shows a stone destroying catheter which has a shroud/guide means which is capable of expanding and contracting. This shroud/guide comprises a plurality of prongs each of which is an extension of a sleeve located between the catheter jacket and a compressor sleeve. U.S. Patent 5,135,483 describes a catheter designed specifically for diagnosis and treatment of life-threatening acute pulmonary thrombosis. The tip of the catheter is surrounded by a shroud which is affixed to the working head of a 2,7 mm (8 French) flexible catheter jacket. The preamble of claim 1 is based on the disclosure of said document.

Concerns have been raised about the safety associated with using the multi-component shroud and catheter system as set forth in the prior art. Due to the typical sizes for a device of this nature, a multi-component shroud could easily come detached from the catheter assembly during use. If this should happen it could be life or limb threatening and require emergency corrective surgery. Further, there are increased costs associated with the manufacture and assembly of a multi-component system.

It is an object of the instant invention to provide a catheter which comprises a bushing/shroud wherein the shroud portion which is rigidly affixed to and extending from the bushing portion thus comprising a single element in the assembly of the catheter as characterized in claim 1.

Our invention introduces a catheter having a rotatable tip which is essentially encompassed by a shroud wherein the shroud fixedly extends from the bushing sleeve thereby providing a bushing/shroud. The design of the bushing/shroud component of the instant invention provides a safer shrouded catheter.

The catheter of the instant invention includes an elongated flexible sheath having a catheter lumen and having a length which extends from outside a subject to a region of interest within a subject vessel. A rotatable tip is driven by a drive means which extends through the catheter lumen. A bushing/shroud is interposed between the flexible sheath and the rotatable tip. The shroud portion extends from the distal end of the bushing portion wherein said shroud essentially surrounds the rotatable tip, the rotatable tip may or may not extend beyond the distal end of the shroud.
FIG. 1 is a side view of the distal end of a catheter constructed in accordance with the present invention.
FIG. 2 is a partially sectioned view of the distal end of a catheter constructed in accordance with the present invention taken along line 2-2 of FIG. 1.
FIG. 3 is a cross sectional view of the bushing/shroud that supports and encompasses a distally located rotatable tip shown in FIG. 2.
FIG. 4 is a cross sectional view of the bushing/shroud taken along line 4-4 of FIG. 3.
FIG. 5 is a three dimensional, exploded, perspective view of the distal subassembly, showing the bushing/shroud, tip and weld collar.
FIG. 6 is a three dimensional perspective view of the distal subassembly as it is assembled.

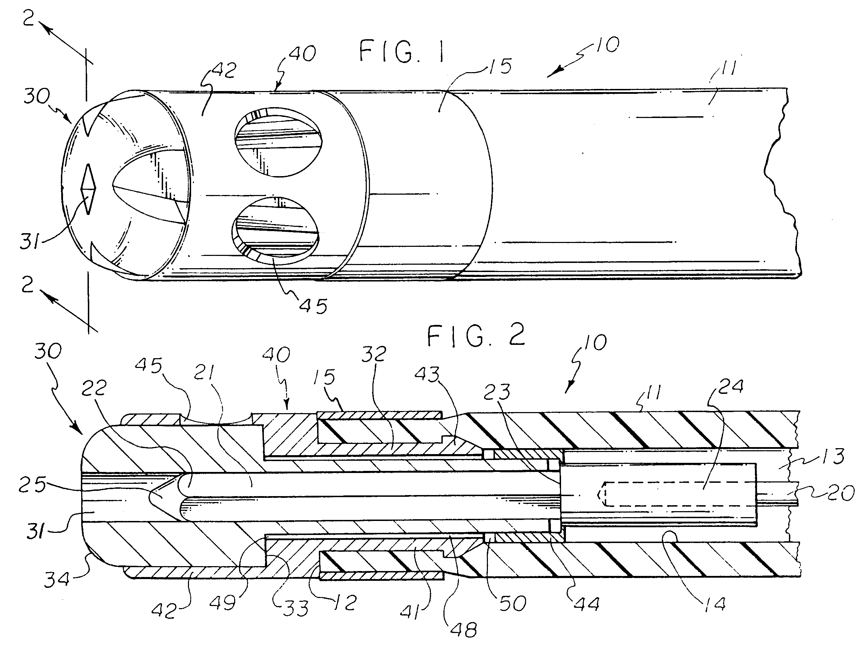

Referring to various figures of the drawings, wherein like reference characters refer to like parts, there is shown in FIGS. 1 and 2 the distal end of a catheter 10 for intravascular or other surgical applications. Catheter 10 comprises an elongated jacket 11 having opposed proximal (not shown) and distal ends 12, defining a catheter lumen 13 extending between and interconnecting the ends. The jacket 11 is formed of suitable material such as plastic and has an outside diameter that is small enough that the catheter 10 can be routed through a vessel to the region needing treatment. Representative sizes of the catheter's outside diameter are 2.7 mm (8 French), 2.2 mm (6.5 French), 1.7 mm (5 French), these sizes are merely exemplary. Thus, in accordance with this invention, the catheter can be constructed as large as 5.0 mm (15 French) and small as 0.67 mm (2 French).

The tip 30 is rotated at high speeds by an associated drive means. Although any suitable drive means may be used, Figure 2 exemplifies the preferred means. As seen in Figure 2, a multi strand flexible drive cable 20 extends through the catheter lumen 13 of catheter 10 and has a drive pin 21 welded to the drive cable 20. The drive pin 21 is engaged with the tip 30 via keyed-through hole 31. The drive cable 20 and drive pin 21 are removable from the catheter lumen 13 of the jacket 11. The drive pin 21 of drive cable 20 has a shoulder 23 for abutting the end of the tip shaft 32 which is integral with the tip 30. Drive pin 21 has an longitudinal hole 24 into which an end of drive cable 20 is attached by a weld. The drive pin 21 of drive cable 20 has a radius apex 25 extending partially into the keyed-through hole 31 of tip 30. Drive pin 21 has an irregular, preferably rectangular traverse cross section, which mates with that of keyed-through hole 31, such that drive pin 21 of drive cable 20 transmits torque to the keyed-through hole 31 for rotating the tip 30 at high speeds.

The preferred means for supporting the drive cable 20 in a central position in the catheter lumen 13 of catheter 10 is by means of a spiral bearing which is common to the art and therefore is not shown herein for clarity of the shown parts. The bearing comprises a helical or spiral cylindrical coil of wire surrounding the drive cable 20. The spiral bearing extends substantially the entire length of the catheter 10 from a proximally located point adjacent the drive motor, which is common to the art and therefore is not shown herein for clarity of the shown parts, to an area just proximal of the drive pin 21. The outer diameter of the helical bearing coils loop clear of the interior surface 14 of jacket 11. The bearing is held securely in place by a stake at the proximal end of the catheter. An inside diameter of a central passageway extending down the length of the helically coiled bearing is slightly greater than the outside diameter of the drive cable 20 so that the drive cable 20 can freely rotate therein. With such a construction, the drive cable 20 can be rotated at a high rate of speed, for example, from 10,000 to 200,000 rpm, while the catheter 10 is bent through a small radius of curvature such as 1.9 cm (0.75 inches).

The tip 30 is located within the bushing/shroud 40 at the distal end 12 of the jacket 11. The tip 30 is arranged to be rotated at high speed with respect to the catheter 10 by the drive means to effect the surgical procedure to be carried out by the catheter 10. The proximal end of the drive cable 20 is located outside the patient's body and is connected to a source of rotary power, e.g. an electric motor (not shown).

At the distal end of the catheter 10 there is secured a bushing/shroud 40. The bushing/shroud 40 comprises a bushing portion 41 for supporting the tip 30 and a shroud portion 42 surrounding the tip 30. The tip 30 has a keyed-through hole 31 which alternatively receives the keyed drive pin surface 21 of the drive cable 20 to rotate the tip 30. When the drive cable 20 and drive pin 21 are withdrawn from the catheter lumen 13, a guidewire or other auxiliary item of correct size (not shown) may be passed through the keyed-through hole 31. The bushing portion 41 is pressed into place axially on the distal end 12 of jacket 11. Locking barb 43 aids in the attachment to the internal surface 14 of jacket 11. A weld collar 44 extends proximally from locking barb 43 to an area just past the proximal end of the tip shaft 32, which in combination with the distal end of the drive pin 22 and the weld collar 44 helps guide the keyed drive pin 21 into the keyed-through hole 31 of tip 30. The configuration of the bushing/shroud 40 of the instant invention allows the shroud portion 42 as well as the bushing portion 41 to be secured into the catheter by a single retention band 15.

Although the instant invention may be used to disintegrate or otherwise destroy particles in any body cavity which can be accessed radiologically or percutaneously, it is particularly suited for the diagnosis and treatment of life or limb-threatening atheroma and thrombosis. Referring to Figures 1, 2 and 6, the tip 30 functions as a high-speed rotary impeller which functions to pulverize atheroma and/or homogenize thrombus. The tip 30 has a rounded shape and is surrounded by the shroud portion 42 extending from the bushing portion 41. As seen in FIGS. 1, 2 and 6 the tip 30 protrudes slightly distal past the shroud portion 42. Prototypes have been made where the shroud portion 42 extends slightly distal of tip 30. In both designs the thrombus or particles are drawn into the tip 30 via the vortex created during high speed tip 30 rotation and are broken up quickly then exhausted through the openings 45 of the shroud portion 42, thereby functioning as a pump/blender. The shroud portion 42 is designed to present a smooth exterior to the vessel lumen, is of relatively thin metal, with smooth edges which shield the vascular tissue from the tip 30 and avoid trauma to the vessel wall. A guide wire (not shown) can be used for tracking the catheter through a vessel lumen by passing the guide wire (not shown) through the keyed-through hole 31 of the tip 30 after the drive cable 20 along with the drive pin 21 has been withdrawn from the catheter lumen 13.

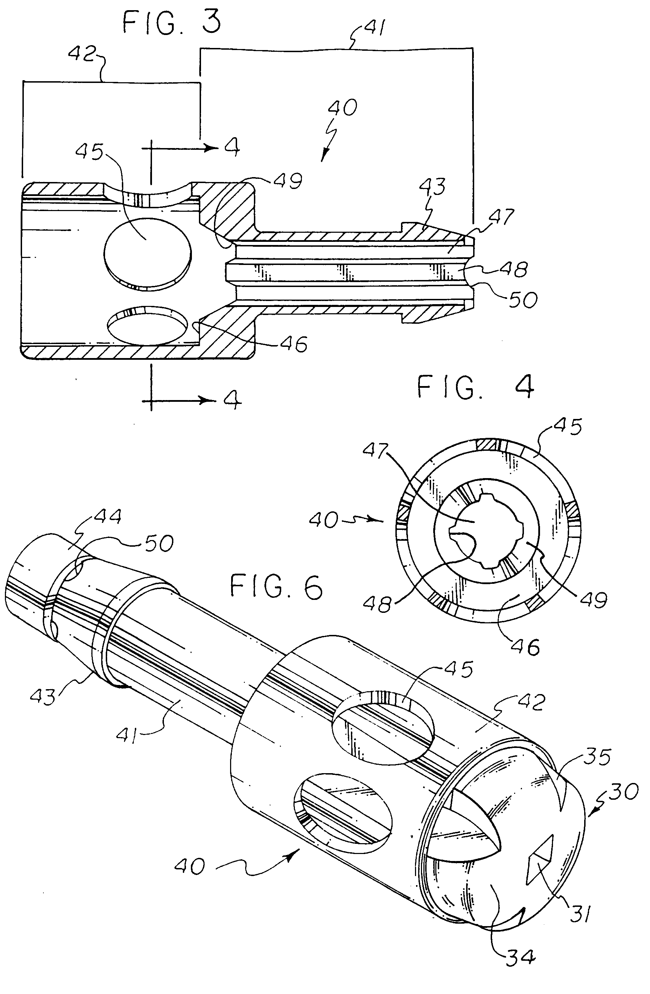

Referring to FIGS. 2 and 3 the bushing/shroud comprises a bushing portion 41 and a shroud portion 42. The planer surface created by the difference between the major and minor diameters of the bushing portion 41, is butted against the distal end 12 of the catheter's jacket 11. The outside diameter of the bushing sleeve portion 41 is approximately that of the inside diameter of the catheter's jacket 11 so that it snugly fits therein. The bushing/shroud 40 is held firmly in place by a retention band 15 which tightly encircles the periphery of the catheter jacket 11. Locking barb 43 which extends about the periphery of the bushing portion 41 digs into the interior surface of the catheter jacket 14 and holds the bushing/shroud tightly in place. Extending from the distal end of the catheter jacket 11 and surrounding the tip 30 is the shroud portion 42 of the bushing/shroud 40. The inside diameter of the shroud portion 42 is greater than the outside diameter of the tip 30 such that a gap or space is formed in between. The shroud portion 42 of the bushing/shroud 40 has a plurality of circular openings 45, although longitudinal or oval openings could be used. In the preferred embodiment of the instant invention, the shroud portion 42 has five equally spaced circular openings 45.

Referring to FIGS. 2 and 4, there are extending down the bore 47 of the bushing portion 41 of the bushing/shroud 40, four, equidistantly spaced, longitudinal grooves 48. The distal end of each longitudinal groove 48 terminates at a fluid exit port 49 located at the distal end of the bushing portion 41, while the proximal end of each longitudinal groove 48 terminates in a respective, generally radially extending, relief groove 50. As seen in FIG. 4, the longitudinal grooves 48 have a generally trapezoidal shape. A fluid may pass down the interior of the catheter lumen 13 of catheter 10, under pressure, into the relief grooves 50, through the associated longitudinal grooves 48 and out through the exit ports 49 at the bushing thrust face 46 of the catheter 10. A liquid can be expelled through the distal subassembly, for example, to aid in dilation of the arterial tissue at the working head. This liquid also serves to cool and lubricate the rotating members of the catheter 10. Moreover, a liquid can be passed down the catheter which is oxygenated to eliminate distal ischemia during the restriction opening procedure by the catheter. Also, if desired, nitrates, contrast media, lytic therapy or other drugs can be delivered as needed during the procedure.

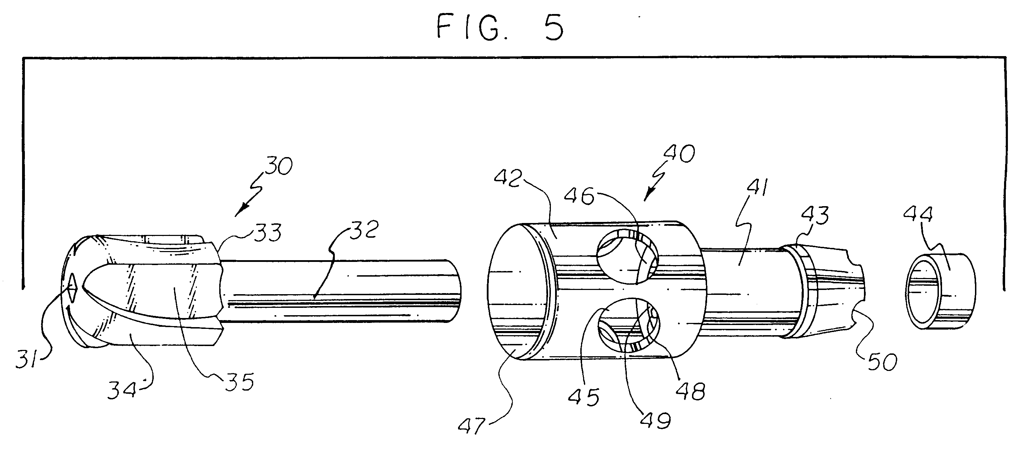

Referring to Figures 2 and 5-6, the tip 30 includes a shaft 32. The tip shaft 32 projects proximally and passes through the bore 47 in the bushing/shroud 40. The tip 30 has a generally planer rear surface 33 which rotates next to the bushing thrust face 46 of the bushing portion 41. The tip 30 is prevented from axial movement within the bushing/shroud 40 by virtue of a weld collar 44 mounted on the proximal end of the tip shaft 32 contiguous with the proximal end of the bushing/shroud 40. FIG. 5 exemplifies the preferred design of the tip 30. The preferred design of the tip 30 basically comprises a solid bodied element whose outer surface 34 is of generally convex shape and which is provided with one or more right hand flutes 35. Each flute 35 has a zero to positive angle rake and a right hand helix or semi helical tooth face. The center of tip 30 has a longitudinal keyed-through hole 31 which allows for the interface of the keyed drive pin 21 and/or passage of other auxiliary devices.

When the catheter 10 is used for treating occlusive atherosclerotic disease, such as opening a restriction in an artery formed by atherosclerotic plaque, the catheter is introduced into the vascular system of the patient such as through an opening in the femoral artery at a point in the groin. The catheter is then guided to the vascular occlusion or blockage that has been determined to exist so that its tip 30 is located immediately adjacent to the restriction.

As recognized by those skilled in the art, arterial restrictions are formed by the deposit of atherosclerotic plaque, fatty steak or other material(s), such as waxy or calcified atheroma and thickened or ulcerated intima. Once in position, the catheter 10 is arranged to translumenally recanalize the diseased artery by ablating, pulverizing and/or cutting the stenotic or occluded area (which may or may not be covered by fibrous plaque) and selectively removing calcified, thrombotic or fatty tissue exposed to the catheter's mechanical action while allowing the artery wall to remain in tact.

## Claims

1. A catheter (10) comprising
(a) an elongated flexible jacket (11) having opposed proximal and distal (12) ends and a catheter lumen (13) extending between the ends,
(b) a rotatable tip (30) located at the distal end (12) of the jacket (11),
(c) a drive means (20) extending through the catheter lumen (13) having a drive pin (21) that is removable from the catheter lumen (13) and rotatable tip (30), and
(d) a bushing/shroud (40) supporting and encompassing the rotatable tip (30); defining a bushing portion (41) supporting the rotatable tip (30); and extending into the distal end (12) of the jacket (11) and having a bore to accommodate a portion of the rotatable tip (30) and a shroud portion (42) encompassing the rotatable tip (30),
characterized in that the bushing/shroud is single bodied.

2. A catheter as claimed in claim 1 wherein the shroud portion (42) of said bushing/shroud (40) has a plurality of openings (45).

3. A catheter a claimed in claim 2 wherein the openings (45) are circular.

4. A catheter as claimed in claim 1 wherein the tip (30) comprises a solid bodied element whose outer surface (34) is of generally convex shape and has one or more right hand flutes (35).

5. A catheter as claimed in claim 4 wherein each flute (35) has a zero to positive angle rake and a right hand helix.

6. A catheter as claimed in claim 4 wherein each flute (35) has a zero to positive angle rake and a semi helical tooth face.

## Patentansprüche

1. Katheter (10) enthaltend
(a) eine längliche flexible Ummantelung (11) mit einander gegenüberliegenden proximalen und distalen Enden (12) und einem sich zwischen den Enden erstreckenden Katheterlumen (13),
(b) eine drehbare Spitze (30), die an dem distalen Ende (12) der Ummantelung (11) angeordnet ist,
(c) ein Antriebsmittel (20), das sich durch das Katheterlumen (13) hindurch erstreckt, mit einem Antriebsstift (21), der aus dem Katheterlumen (13) heraus und von der drehbaren Spitze (30) abnehmbar ist, und
(d) eine Laufbuchse/Abdeckung (40), die die drehbare Spitze (30) trägt und umfaßt; wobei sie einen die drehbare Spitze (30) tragenden Laufbuchsenteil (41) definiert und sich in das distale Ende (12) der Ummantelung (11) erstreckt und ein Loch aufweist, um einen Teil der drehbaren Spitze (30) aufzunehmen und einen Abdeckungsabschnitt (42), der die drehbare Spitze (30) umfaßt,
dadurch gekennzeichnet, daß die Laufbuchse/Abdeckung einteilig ist.

2. Katheter wie in Anspruch 1 beansprucht, bei dem der Abdeckungsabschnitt (42) der Laufbuchse/Abdeckung (40) eine Mehrzahl von Öffnungen (45) aufweist.

3. Katheter wie in Anspruch 2 beansprucht, bei dem die Öffnungen (45) kreisförmig sind.

4. Katheter wie in Anspruch 1 beansprucht, bei dem die Spitze (30) ein Massivkörperelement enthält, dessen Außenfläche (34) eine im wesentlichen konvexe Form aufweist und ein oder mehrere rechtssteigend verlaufende Riefen (35) aufweist.

5. Katheter wie in Anspruch 4 beansprucht, bei dem jede Riefe (35) einen Steigungswinkel von Null Grad bis zu positiven Werten und eine rechtssteigend verlaufende Wendelung aufweist.

6. Katheter wie in Anspruch 4 beansprucht, bei dem jede Riefe (35) einen Steigungswinkel von Null Grad bis zu positiven Werten und eine Halbschrägverzahnungsoberfläche aufweist.

## Revendications

1. Cathéter (10) comprenant
(a) une gaine souple allongée (11) présentant des extrémités opposées proximale et distale (12) ainsi qu'une lumière de cathéter (13) s'étendant entre les extrémités,
(b) une pointe rotative (30) placée à l'extrémité distale (12) de la gaine (11),
(c) un moyen d'entraînement (20) s'étendant à travers la lumière de cathéter (13) comportant une broche d'entraînement (21) qui peut être enlevée de la lumière de cathéter (13) et de la pointe rotative (30), et
(d) une douille/protection (40) supportant et entourant la pointe rotative (30) ; définissant une partie de douille (41) qui supporte la pointe rotative (30) et s'étend dans l'extrémité distale (12) de la gaine (11) et comportant un alésage destiné à recevoir une partie de la pointe rotative (30) ainsi qu'une partie de protection (42) entourant la pointe rotative (30),
caractérisé en ce que la douille/protection est en un seul corps.

2. Cathéter selon la revendication 1, dans lequel la partie de protection (42) de ladite douille/protection (40) comporte une pluralité d'ouvertures (45).

3. Cathéter selon la revendication 2 dans lequel les ouvertures (45) sont circulaires.

4. Cathéter selon la revendication 1 dans lequel la pointe (30) comprend un élément en un seul corps dont la surface extérieure (34) est de forme générale convexe et comporte une ou plusieurs gougures à droite (35).

5. Cathéter selon la revendication 4 dans lequel chaque gougure (35) présente un angle de coupe de nul à positif et une hélice à droite.

6. Cathéter selon la revendication 4 dans lequel chaque gougure (35) présente un angle de coupe de nul à positif et une face de dent semi-hélicoïdale.
